# EUROPEAN PATENT SPECIFICATION

(11) **EP 1 839 627 B1**
(45) Date of publication and mention of the grant of the patent: **12.01.2011**
(21) Application number: 07251332.8
(22) Date of filing: 28.03.2007
(51) Int. Cl.: A61F 2/38

(54) **A knee joint prosthesis component**
Komponente einer Kniegelenkprothese
Composant de genou prothétique

(30) Priority: 31.03.2006 US 395617
(43) Date of publication of application: 03.10.2007
(73) Proprietor: DePuy Products, Inc., Warsaw, IN 46581 (US)
(72) Inventor: Wagner, Christel M., Plymouth, IN 46563 (US); Auger, Daniel D., Fort Wayne, IN 46814 (US)
(74) Representative: Belcher, Simon James

(56) References cited:
- WO-A-03/007787
- FR-A1- 2 738 739
- US-A- 6 056 777
- US-A1- 2005 267 584

## Description

This invention relates to the field of arthroplasty, and particularly to a knee joint prosthesis component.

Arthroplasty involves the surgical reconstruction or replacement of a malformed or degenerated joint. With interpositional arthroplasty, a knee implant is placed between inflamed joint surfaces to keep them apart. Such knee implants are often referred to as knee spacers.

In one common interpositional arthroplasty procedure, a knee spacer is placed between the tibia and the femur. In particular, the knee spacer is implanted between either the medial or lateral condyle of the femur and the meniscus of the tibia. The knee spacer provides a surface for articulation of the femur relative to the tibia.

Knee spacers are generally designed to conform to either the femur or the tibia in an attempt to prevent dislocation of the knee spacer within the joint. Various methods have been proposed for conforming the knee spacer within the joint. For example, some knee spacers are formed with a posterior lip that extends distally over the tibia. Other knee spacers are designed to conform to the femur in an attempt to retain the spacer within the joint. However, it has been noted that many of these knee spacers do not prevent in vivo movement of the knee spacer. In vivo movement of the knee spacer is one factor that may significantly contribute to the pain a patient experiences following an interpositional arthroplasty procedure.

US-A-2005/0267584 discloses tibial components of knee joint prosthesis devices which can be provided with an anchor in the form of an elongate keel, which can be fitted into a slot in the tibia to locate the resected tibia.

It would be advantageous to provide a knee spacer for a knee joint prosthesis component that may be secured in such a manner to prevent in vivo movement of the knee spacer relative to the femur or tibia. It would be of further advantage if such knee spacer could be secured to the tibia in a manner that does not violate the subcondylar plate. It would also be advantageous if the knee spacer could be easily fixed to the femur or the tibia.

Accordingly, the invention provides an interpositional spacer for attachment to the surface of a patient's tibia for articulation with the natural bearing surface of the femur, as defined in claim 1.

The implant of the invention can be used in a method of providing a knee joint prosthesis component comprising:
(a) providing a spacer having at least one fastener extending from the spacer;
b) forming at least one hole in a condyle, the at least one hole extending through articular cartilage and into a subcondylar plate;
(c) attaching the spacer to the condyle by inserting at least one fastener into the at least one hole.

In operation, a surgeon forms one or more holes in the tibial condyle. In one example, the holes do not perforate the subcondylar plate that forms the perimeter portion of the subcondylar bone. In this example, the holes extend through the articular cartilage and into the subcondylar plate, but do not extend completely through the subcondylar plate. In an alternative example, the holes formed by the surgeon extend completely through the subcondylar plate.

After the holes are formed in the condyle, the surgeon orients the spacer on the condyle with the fasteners directed toward the holes. The surgeon then presses against the spacer, forcing the fasteners into the holes. As the fasteners are forced into the holes, the deformable fins collapse. The collapsed fins act to wedge the pegs in the holes, thus securing the spacer to the condyle.

Embodiments of the invention will now be described by way of example with reference to the accompanying drawings, in which:
FIG. 1 shows a top view of a knee joint prosthesis component;
FIG. 2 shows a fastener for the knee joint prosthesis component of FIG. 1;
FIG. 3 shows a bottom view of the knee joint prosthesis component of FIG. 1;
FIG. 4 shows a side view of the knee joint prosthesis component of FIG. 1;
FIG. 5 shows a cross-sectional view of the knee joint prosthesis component along line V-V of FIG. 4;
FIG. 6 shows a perspective view of a femur and tibia with a first interpositional knee arthroplasty of FIG. 1 attached to the lateral plateau of the tibia and a second interpositional knee arthroplasty attached to the medial plateau of the tibia;
FIG. 7 shows a proximal view of the tibia of FIG. 6 showing the first interpositional knee arthroplasty positioned in the lateral plateau and the second interpositional knee arthroplasty positioned in the medial plateau; and
FIG. 8 shows a cross-sectional view of the tibia of FIG. 6 showing holes formed in the tibial condyles and the peg members extending into the subcondylar plate of the tibia.

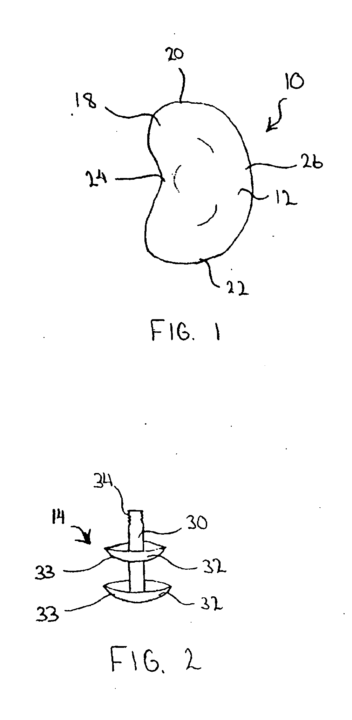

Referring to the drawings, FIGs. 1 to 5 show a knee joint prosthesis component 10 which comprises a spacer 12 and a plurality fasteners 14 extending from the spacer 12. As explained herein, the arthroplasty 10 is configured for attachment to either the lateral or medial plateau of the tibia.

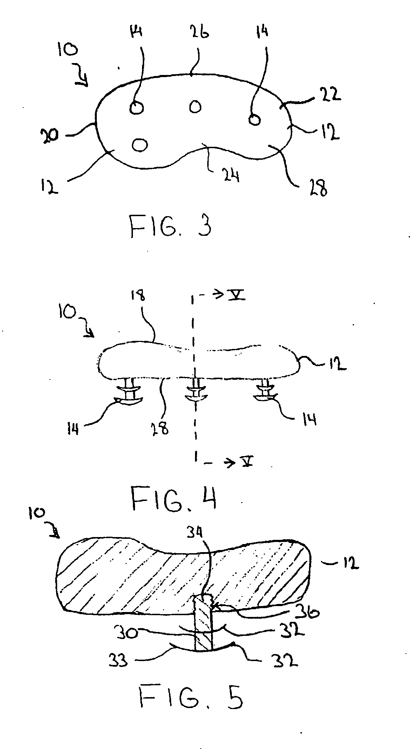

In the embodiment shown in FIGs. 1 and 3, the spacer 12 comprises a plate that is generally kidney shaped when viewed from the top and bottom. The spacer 12 is greater in length measured from its anterior side 20 to its posterior side 22 than it is in width measured from its medial side 24 to its lateral side 26. While the kidney shape of the spacer has advantages with respect to attachment of the spacer to the condyle, one of skill in the art will recognize that other shaped spacers may also be used.

The spacer 12 is comprised of a biocompatible material, either a polymer or metal such as ultra high molecular weight polyethylene (UHMWPE), polyurethane (PU), cobalt chrome (CoCr), or titanium (Ti). As shown in FIGs. 1 and 4, the top surface 18 of the spacer 12 is generally smooth with rolling contours. These rolling contours are designed to mimic the surface of a condyle facing the meniscus of the tibia.

As shown in FIGs. 3 and 4, the bottom surface 28 of the spacer 12 is generally flat with the fasteners 14 extending from the bottom surface 28. In one embodiment, the spacer 12 is somewhat flexible, allowing the bottom surface 28 to be bent in order to match the curved surface of the condyle when the spacer 12 is attached to the condyle. This distortion of the spacer 12 to match the curved surface of the condyle is more easily achieved because of the kidney shape of the spacer. In particular, the kidney shape of the spacer facilitates spacer distortion such that the bottom surface 28 more closely conforms to the curved surface of the condyle. In an alternative embodiment, the spacer 12 is more rigid, but the bottom surface 28 of the spacer is curved, allowing the bottom surface 28 of the spacer to conform to the curved surface of the condyle.

With reference to FIG. 4, the fasteners 14 may comprises peg members 15 that extend from the bottom surface 28 of the spacer 12. The plurality of peg members 15 each include a rigid centre peg 30 and a plurality of deformable fins 32 attached to the centre peg. In the disclosed embodiment, the deformable fins 32 comprise cup structures 33. Each cup structure 33 is secured at a central location to the centre peg 30 and extends from such central location upward toward the bottom surface 28 of the spacer 12. A first cup structure 33 is positioned at the end of the centre peg 30. A second cup structure is positioned at a midpoint of the centre peg.

The cup structures 33 are secured to the centre peg 30 by any of numerous methods. For example, the cup structures 33 may be secured to the centre peg using adhesives or fasteners. In one embodiment, the centre peg is comprised of a plurality of peg segments which are attached by one peg segment which engages an adjacent peg segment by means of a thread. In this arrangement, an extending screw portion of one peg segment is inserted through a centre hole in a cup member and the adjacent peg sections are screwed together. This action clamps the centre hole of the cup member between peg segments. Of course, numerous other methods may be used to secure the cup members 33 to the centre peg, as will be recognized by those of skill in the art.

The centre peg member is comprised of an appropriate rigid bio-compatible material, either a polymer or metal such as titanium or cobalt chromium. As shown in FIG. 5, each centre peg 30 extends into the spacer 12 through the bottom surface 28, allowing the centre peg to be fixed to the spacer. The centre peg includes a threaded top portion 34. The threads on the top portion 34 of the centre peg 30 engage threaded holes 36 in the bottom of the spacer to secure the centre peg to the spacer. Adhesives may be used in the holes 36 of the spacer 12 to further secure the peg members 14 to the spacer 12. Of course, various other methods may be used to secure the centre posts to the holes in the spacer, such as adhesives, friction fit, or snap fit arrangements, as well as other arrangements as will be recognized by those of skill in the art. Alternatively, the peg members may be integral with the spacer, such as an arrangement where the peg members are moulded with the spacer as a one piece construction.

The deformable fins 32 or cup members 33 are designed in a manner and/or are comprised of an appropriate material that facilitates deformation of the fins. For example, the deformable fins 32 may be comprised of an appropriate deformable biocompatible material having relatively flexible characteristics, such as polyethylene or polyurethane. As explained in further detail below, the deformable cup members 33 are designed to collapse when forced into holes formed in the articular cartilage of the condyle. In alternative embodiments, the deformable fins 32 take different shapes other than that of cup members. For example, the deformable fins may comprise clover structures, spoked structures, circular structures that are not cupped, or numerous other designs. Furthermore, with certain designs, the deformable fins 32 may be comprised of a relatively rigid material instead of a flexible material. For example, in one embodiment, the deformable fins are comprised of a relatively rigid metal material arranged as a clover leaf cup structure. Such rigid metal fin structures will typically have a lesser thickness than fins comprised of more flexible material such as PE or PU.

With reference to FIG. 3, the plurality of peg members 15 are placed on the bottom surface 28 of the spacer 12 in an arrangement that promotes a secure attachment of the spacer 12 to the condyle. In the arrangement of FIG. 3, four peg members 15 are provided on the bottom surface 28 of the spacer 12. However, depending on numerous factors such as the size of the patient and particular shape of the spacer, different numbers of peg members may be used in different configurations.

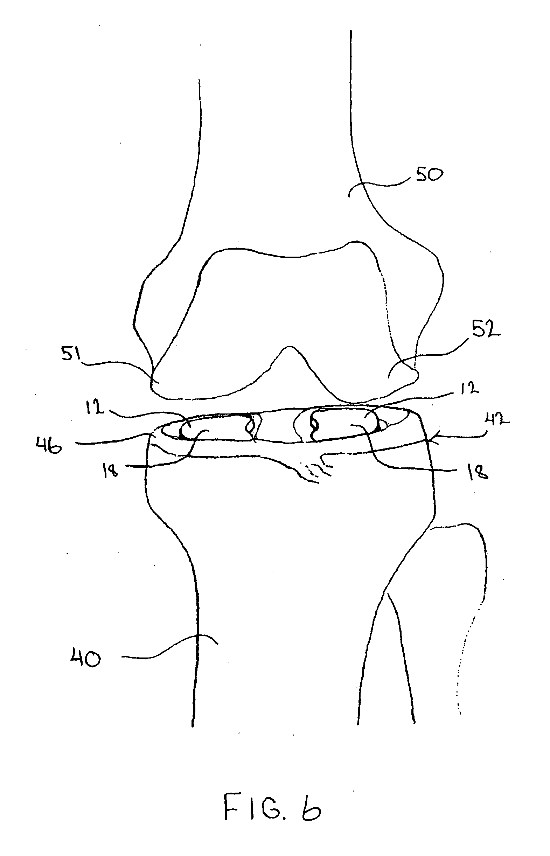

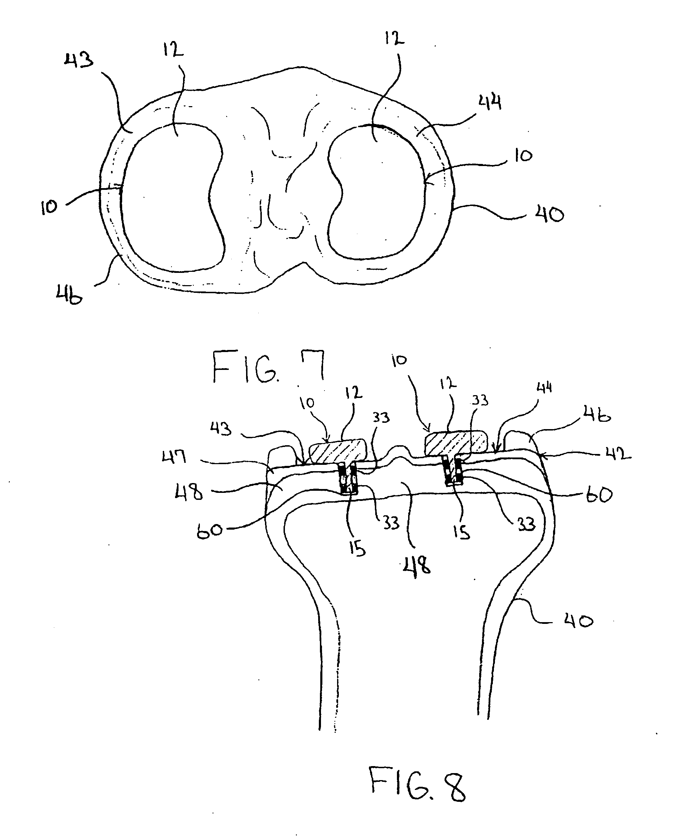

In order to secure the interpositional arthroplasty to a tibial condyle, the surgeon first prepares the condyle by forming holes in the condyle. FIGs. 6-8 show an exemplary embodiment where an interpositional arthroplasty 10 is secured to each tibial condyle 43 and 44. FIG. 8 particularly shows a cross-sectional view of the tibia 40 with holes 60 formed in the tibial condyles 43 and 44.

Before the holes 60 are formed in the condyles 43 and 44, the surgeon first clears the interior portion of the meniscus 46. With the interior of the meniscus 46 cleared, the medial tibial condyle 43 and lateral tibial condyle 44 are open to receive an arthroplasty 10. In one embodiment, the surgeon may also smooth the exterior articular cartilage 47 to prepare a surface for the spacer 12.

Once the interior portion of the meniscus 46 is cleared, holes 60 are formed in the condyles 43 and 44 using a drill and guide arrangement. Such arrangements are common in prosthetic procedures. In this case, the drill and guide arrangement is configured to create a hole 60 in the condyle that extends to the subcondylar plate 48. In one embodiment, the depth of the hole extends through the articular cartilage 47, but does not extend completely through the subcondylar plate 48. The actual depth of such a hole will depend upon the patient, but the typical depth of such a hole in the condyle that does not extend through the subcondylar plate 48 is less than eight mm. In an alternative embodiment, the hole created by the surgeon in the condyle extends completely through the subcondylar plate 48, thus perforating the subcondylar plate. In any event, the diameter of the hole that is created in the condyle is slightly larger than the diameter of the centre peg of a peg member, but smaller than the diameter of deformable a cup member 33. This allows the peg member to be inserted into the hole while causing the cup members 33 to collapse.

After the holes are formed in the condyles, the surgeon aligns the peg members 15 with the holes in the condyle and manually presses against the spacer 12 to force the peg members 15 into the holes. FIG. 8 shows the peg members 15 inserted in the holes 60. When the deformable cup members 33 are forced into the holes in the condyle along with a peg member 15, the deformable cup members 33 collapse since the diameter of the hole 60 is less than the diameter of the cup members 33. The deformed cup members 33 are collapsed into excessive space in the hole and wedge the centre peg in the hole in a friction fit arrangement. The deformed cup members are represented in FIG. 8 by the rectangular portions positioned between the holes 60 and the peg members 15. The wedging action of the deformed cup members effectively secures the arthroplasty to the associated condyle.

Following implantation, the spacer 12 of each arthroplasty 10 is secured to a condyle 43 or 44 and covers a substantial portion of the exterior surface of the condyle. In particular, each spacer 12 is configured to cover the articular cartilage 47 surface portion of the condyle that would normally contact the meniscus 46 of the tibia 44. Thus, as shown in FIG. 6, the top surface 18 of each spacer 12 is exposed to the femur 50, with each top surface 18 directly opposed to a femoral condyle 51, 52. With the spacers 12 in place, inflamed joint surfaces on the femur and tibia are separated, thus relieving pain encountered by the patient. Furthermore, with the arthroplasty secured to the condyle, in-vivo movement of the arthroplasty is restricted, and less pain is encountered by the patient following the procedure. Also, because the arthroplasty may be secured without perforating the subcondylar plate, the patient may experience less pain following surgery along with a faster recovery time.

While FIGs. 6-8 shown a knee joint prosthesis component 10 attached to both the medial tibial condyle 43 and the lateral tibial condyle 44, some surgeries may only call for the use of a single arthroplasty secured to a single condyle, depending on the needs of the patient.

## Claims

1. An interpositional spacer (12) for attachment to the surface of a patient's tibia for articulation with the natural bearing surface of the femur, in which the spacer can flex so that its bottom surface (28) can match the curved surface of the tibial condyle when the spacer (12) is attached to the condyle, **characterised in that** the spacer has a plurality of fasteners (14) which extend from its bottom surface so as to extend through separate holes in the articular cartilage and into the subcondylar plate, each of the fasteners comprising a peg (30) having at least one deformable fin (32) on it.

2. The spacer of claim 1 in which the spacer (12) comprises a kidney shaped plate.

3. The spacer of claim 1 in which the deformable fin (32) comprises a cup member.

## Patentansprüche

1. Zwischenliegender Abstandhalter (12) zum Anbringen an der Oberfläche eines Schienbeins eines Patienten zur Gelenkbildung mit der natürlichen Lagerfläche des Oberschenkels, wobei sich der Abstandhalter so biegen kann, dass sich seine Bodenfläche (28) an die gebogene Fläche der tibialen Kondyle anpassen kann, wenn der Abstandhalter (12) an der Kondyle angebracht ist, **dadurch gekennzeichnet, dass** der Abstandhalter eine Vielzahl von Befestigungselementen (14) hat, die sich von seiner Bodenfläche erstrecken, so dass sie sich durch getrennte Löcher in den Gelenkknorpel und in die Subkondylenplatte erstrecken, wobei jedes der Befestigungselemente einen Stift (30) mit wenigstens einer deformierbaren Lamelle (32) darauf aufweist.

2. Abstandhalter nach Anspruch 1, wobei der Abstandhalter (12) eine nierenförmige Platte aufweist.

3. Abstandhalter nach Anspruch 1, bei dem die deformierbare Lamelle (32) ein Becherelement aufweist.

## Revendications

1. Entretoise d'interposition (12) destinée à une fixation sur la surface d'un tibia d'un patient et une articulation avec la surface d'appui naturelle du fémur, dans laquelle l'entretoise peut fléchir de telle sorte que sa surface inférieure (28) puisse s'adapter à la surface incurvée du condyle tibial lorsque l'entretoise (12) est fixée au condyle, **caractérisée en ce que** l'entretoise présente une pluralité d'attaches (14) qui s'étendent à partir de sa surface inférieure de manière à s'étendre à travers des trous séparés dans le cartilage articulaire et dans la plaque sous-condylienne, chacune des attaches comprenant une cheville (30) qui présente au moins un ergot déformable (32) situé dessus.

2. Entretoise selon la revendication 1, dans laquelle l'entretoise (12) comprend une plaque en forme de rein.

3. Entretoise selon la revendication 1, dans laquelle l'ergot déformable (32) comprend un élément de coupelle.
